Europäisches Patentamt

European Patent Office

Office européen des brevets

(11) Numéro de publication : **0 384 854 B1**

## (12) FASCICULE DE BREVET EUROPEEN

(45) Date de publication du fascicule du brevet :
**28.10.92 Bulletin 92/44**

(51) Int. Cl.⁵ : **A61F 2/38**

(21) Numéro de dépôt : **90420060.7**

(22) Date de dépôt : **05.02.90**

(54) **Implant pour genou, à double courbure et/ou à épaisseur dégressive.**

(30) Priorité : **06.02.89 FR 8901850**

(43) Date de publication de la demande :
**29.08.90 Bulletin 90/35**

(45) Mention de la délivrance du brevet :
**28.10.92 Bulletin 92/44**

(84) Etats contractants désignés :
**CH DE FR GB LI SE**

(56) Documents cités :
**FR-A- 2 141 126**
**US-A- 3 946 446**

(56) Documents cités :
**MEDIZINISCH-ORTHOPÄDISCHE TECHNIK,
vol. 101, no. 6, novembre-décembre 1981,
pages 169-175, Bad Buchau, DE; W. THO-
MAS:**"**Biomechanische Gesichtspunkte zur
Konstruktion von Kniegelenkendoprothesen**"
**JOURNAL OF THE AMERICAN PHARMACEU-
TICAL ASSOCIATION, vol. NS-17, no. 5, mai
1977, pages 297-300; M. SHERMAN:**
"**Orthopedicimplants**"

(73) Titulaire : **GEBRÜDER SULZER
AKTIENGESELLSCHAFT
Zürcherstrasse 9
CH-8401 Winterthur (CH)**
Titulaire : **Protek AG
Stadtbachstrasse 64
CH-3001 Bern (CH)**

(72) Inventeur : **Broc, Christian
Les Baumettes
F - 84220 Gordes (FR)**

## Description

L'invention concerne un implant fémoral pour prothèse bi-compartimentaire d'articulation du genou.

Ce type d'implant a un profil longitudinal en section qui correspond sensiblement au rayon de courbure moyen du condyle. Un implant de ce type est connu du document FR-A-2141126. Il s'avère que ce profil courbe donne entièrement satisfaction.

On sait, par ailleurs, qu'en flexion du genou, l'implant doit glisser sur l'élément tibial.

En ayant pour but de résoudre le problème posé, d'absorber les contraintes de rotation, il est apparu nécessaire que le moment de rotation de la courbure postérieure de l'implant, en flexion, corresponde au moment de rotation de l'arthrose. Or, dans le cas où les plateaux tibiaux sont équilibrés et de même hauteur, l'implant fémoral peut s'avérer, en extension, d'une épaisseur insuffisante.

Pour résoudre un tel problème et être stable en extension, on pourrait envisager d'augmenter l'épaisseur de l'implant fémoral.

Une telle solution n'est pas satisfaisante, car on déplace le moment de rotation de la courbure postérieure de l'implant en flexion, ce qui a pour effet d'augmenter la contrainte en flexion sur le plateau tibial correspondant, lequel a tendance, dans ces conditions, à basculer.

L'invention s'est fixée pour but de remédier à ces inconvénients. Pour résoudre le problème posé de mettre en correspondance le moment de rotation de la courbure postérieure de l'implant en flexion avec celui de l'arthrose, notamment dans le cas de plateaux tibiaux de même hauteur, l'implant fémoral présente, à partir de son extrémité antérieure en extension, un profil longitudinal en section progressivement croissant puis progressivement dégressif, jusqu'à son extrémité postérieure en flexion, qui est d'épaisseur constante.

Un autre problème que se propose de résoudre l'invention est, d'une part, d'avoir en extension, une meilleure répartition et surface d'appui de l'implant fémoral sur le plateau tibial et, d'autre part, d'avoir en flexion, un contact minimum, pour ne pas engendrer une contrainte rotationnelle Un tel problème est résolu en ce que l'implant fémoral, selon l'invention, présente transversalement une double courbure à savoir, très sensiblement dans sa partie médiane, un important rayon de courbure, pour obtenir, en extension, une meilleure répartition et surface d'appui sur un plateau tibial et, au niveau de son extrémité postérieure, un rayon de courbure réduit, pour obtenir en flexion, un contact réduit afin de ne pas distribuer la contrainte rotationnelle.

D'une manière particulièrement avantageuse, l'implant fémoral peut combiner les deux caractéristiques précédemment définies, permettant ainsi, de résoudre chacun des problèmes posés.

En ce qui concerne la fixation de cet implant, qui peut s'effectuer avec ou sans ciment, et pour résoudre le problème d'éviter tout risque de glissement angulaire, l'implant fémoral selon l'invention présente au moins deux plots d'ancrage, dont l'un est situé au niveau de l'extrémité antérieure, tandis que l'autre est situé au niveau de l'extrémité postérieure, la longueur des dits plots (2) et (3) étant déterminée pour affleurer, sans la dépasser, une ligne fictive reliant chacune desdites extrémités.

Un autre problème que se propose de résoudre l'invention, est d'améliorier la fixation de l'implant, notamment dans le cas où ce dernier est posé sans ciment.

Dans ce but, le plot situé du côté de l'extrémité postérieure est incliné par rapport à la verticale, du côté de la dite extrémité.

Avantageusement, l'angle d'inclinaison est de l'ordre de 1 degré.

Enfin, pour éviter l'apparition d'une zone de fatigue, l'implant présente, en combinaison avec les plots et suivant son axe médian, une ailette de rigidité.

L'invention est exposée ci-après, plus en détail, à l'aide des dessins annexés, dans lesquels :

La figure 1 est une vue en coupe longitudinale de l'implant.

la figure 2 est une vue en coupe transversale considérée selon la ligne 2-2 de la figure 1.

La figure 3 est une vue en coupe transversale considérée selon la ligne 3-3 de la figure 1.

Afin de rendre plus concret l'objet de l'invention, on le décrit maintenant d'une manière non limitative en se référant aux exemples de réalisation des figures des dessins.

D'une manière parfaitement connue, l'implant fémoral désigné dans son ensemble par (1) est établi avec un rayon de courbure latéral qui correspond à celui du condyle où il doit être posé.

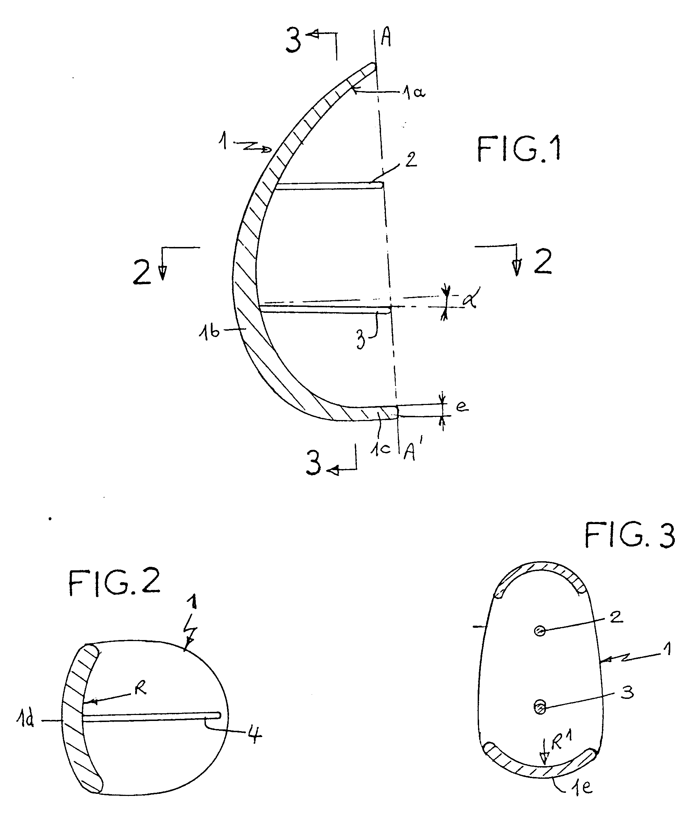

Selon l'invention, l'implant présente, à partir de son extrémité antérieure en extension (1a), un profil longitudinal en section progressivement croissant (1b), puis progressivement dégressif, jusqu'à son extrémité postérieure en flexion (1c) qui est d'épaisseur (e) constante (figure 1).

Très sensiblement, dans sa partie médiane, l'implant (1) présente un profil transversal convexe (1d), dont le rayon de courbure (R) est relativement important pour obtenir, en extension, une meilleure répartition et surface d'appui sur le plateau tibial correspondant (figure 2).

Inversement, au niveau de son extrémité postérieure, l'implant présente un profil latéral convexe (1e), dont le rayon de courbure (R1) est beaucoup plus réduit, afin d'avoir, en flexion, un contact minimum avec le plateau tibial, pour ne pas distribuer la contrainte rotationnelle (figure 3).

Comme le montre la figure 1, l'implant (1) présente au moins deux plots d'ancrage (2) et (3). Le plot (2) est situé du côté de l'extrémité antérieure, tandis que le plot (3) est situé du côté de l'extrémité postérieure. Les plots (2) et (3) sont de largeur différente et déterminée pour affleurer, sans la dépasser, une ligne fictive A-A' reliant chacune des extrémités de l'implant.

Le plot (3) est incliné par rapport à la verticale du côté de l'extrémité postérieure (1c), selon un angle (α), de l'ordre de 1 degré. Une telle disposition améliore la fixation, notamment dans le cas où l'implant est posé sans ciment.

En outre, la face interne de l'implant (1) présente en combinaison avec les plots (2) et (3), une ailette de rigidité (4), apte à éviter toute zone de fatigue.

Les avantages ressortent bien de la description.

## Revendications

1- Implant pour genou du type de ceux dont le profil en section correspond au rayon de courbure moyen du condyle, caractérisé en ce qu'il présente, à partir de son extrémité antérieure en extension (1a), un profil longitudinal en section progressivement croissant (1b), puis progressivement dégressif, jusqu'à son extrémité postérieure en flexion (1c) qui est d'épaisseur constante.

2- Implant selon la revendication 1, caractérisé en ce qu'il présente transversalement une double courbure à savoir, très sensiblement dans sa partie médiane, un important rayon de courbure (R), pour obtenir, en extension, une meilleure répartition et surface d'appui sur un plateau tibial et au niveau de son extrémité postérieure, un rayon de courbure réduit (R1), pour obtenir, en flexion, un contact réduit afin de ne pas distribuer la contrainte rotationnelle.

3- Implant selon l'une quelconque des revendications 1 et 2, caractérisé en ce qu'il présente au moins, deux plots d'ancrage (2) et (3), dont l'un est situé au niveau de l'extrémité antérieure tandis que l'autre est situé au niveau de l'extrémité postérieure, la longueur desdits plots (2) et (3) étant déterminée pour affleurer, sans la dépasser, une ligne fictive, reliant chacune desdites extrémités.

4- Implant selon la revendication 3, caractérisé en ce que le plot (3) situé du côté de l'extrémité postérieure est incliné par rapport à la verticale, du côté de la dite extrémité.

5- Implant selon la revendication 4, caractérisé en ce que l'angle d'inclinaison est de l'ordre de 1 degré.

6- Implant selon l'une quelconque des revendications 4 et 5, caractérisé en ce qu'il présente, en combinaison avec les plots et suivant son axe médian, une ailette de rigidité (3).

## Claims

1. A knee implant of the type in which the profile in section corresponds to the mean radius of curvature of the condyle,
   **characterised in that** it has, from its extended anterior end (1a), a longitudinal profile in section which progressively increases (1b), then progressively decreases, to its bent posterior end (1c), which has a constant thickness.

2. An implant according to Claim 1,
   **characterised in that** transversally it has a double curvature, i.e., very roughly in its median part, a large radius of curvature (R), so as to obtain, during extension, a better distribution and bearing surface on a tibial plate and at the level of its posterior end, a reduced radius of curvature (R1), so as to obtain, during bending, a reduced contact so as not to distribute the rotational stress.

3. An implant according to any one of Claims 1 and 2,
   **characterised in that** it has at least two fixing pins (2) and (3), one of which is situated at the level of the anterior end whereas the other is situated at the level of the posterior end, the length of said pins (2) and (3) being determined so as to be flush with, but not pass beyond, an imaginary line connecting each of said ends.

4. An implant according to Claim 3,
   **characterised in that** the pin (3) situated on the side of the posterior end is inclined with respect to the vertical, on the side of the said end.

5. An implant according to Claim 4,
   **characterised in that** the angle of inclination is in the region of 1 degree.

6. An implant according to any one of Claims 4 and 5,
   **characterised in that** it has, in combination with the pins and along its median axis, a stiffening flange (3).

## Patentansprüche

1. Implantat für ein Knie des Typs, bei dem das Profil im Schnitt dem mittleren Krümmungsradius des Gelenkkopfes entspricht,
   dadurch **gekennzeichnet,**
   daß es ausgehend von seinem im gestreckten Zustand vorderen Ende (1a) bis zu seinem im abgebogenen Zustand hinteren Ende (1c), welches von konstanter Dicke ist, ein Längsschnittprofil (Ib) mit progressiv zunehmender und anschließend progressiv abnehmender Stärke aufweist.

2.  Implantat nach Anspruch 1,
    dadurch **gekennzeichnet**,
    daß es im Querschnitt eine doppelte Krümmung aufweist, nämlich im wesentlichen in seinem Mittelbereich einen großen Krümmungsradius (R), um im gestreckten Zustand eine bessere Druckverteilung und Abstützfläche auf einem Tibiaplateau zu erhalten, und in der Höhe des hinteren Endes einen reduzierten Krümmungsradis (R1), um im abgebogenen Zustand einen reduzierten Kontakt zu erhalten, um die Drehbelastung nicht zu übertragen.

3.  Implantat nach einem der Ansprüche 1 und 2,
    dadurch **gekennzeichnet**,
    daß es zumindest zwei Ankerzapfen (2) und (3) aufweist, von denen einer in Höhe des vorderen Endes angeordnet ist, während der andere in Höhe des hinteren Endes angeordnet ist, wobei die Länge der Zapfen (2) und (3) derart bestimmt ist, um mit einer gedachten, die beiden Enden verbindenden Linie zu fluchten, ohne diese zu überschreiten.

4.  Implantat nach Anspruch 3,
    dadurch **gekennzeichnet**,
    daß der Zapfen (3), der auf der Seite des hinteren Endes angeordnet ist, bezüglich der Vertikalen zur Seite dieses Endes hin geneigt ist.

5.  Implantat nach Anspruch 4,
    dadurch **gekennzeichnet**,
    daß der Neigungswinkel in der Größenordnung von 1° liegt.

6.  Implantat nach einem der Ansprüche 4 und 5,
    dadurch **gekennzeichnet**,
    daß es, kombiniert mit den Zapfen und seiner Mittelachse folgend, eine Versteifungsrippe (3) aufweist.

FIG.1

FIG.2

FIG. 3